# EUROPEAN PATENT SPECIFICATION

(11) **EP 2 701 577 B1**
(45) Date of publication and mention of the grant of the patent: **10.06.2015**
(21) Application number: 12717977.8
(22) Date of filing: 13.04.2012
(51) Int. Cl.: A61B 5/00, H04L 29/08, G06F 3/048, A61N 1/372, G06F 9/38

(54) **PREDICTIVE BACKGROUND DATA TRANSFER FOR IMPLANTABLE MEDICAL DEVICES**
PRÄDIKTIVE HINTERGRUNDDATENÜBERTRAGUNG FÜR IMPLANTIERBARE MEDIZINISCHE VORRICHTUNGEN
TRANSFERT DE DONNÉES D'ARRIÈRE-PLAN PRÉDICTIVES POUR DES DISPOSITIFS MÉDICAUX IMPLANTABLES

(30) Priority: 28.04.2011 US 201113096581
(43) Date of publication of application: 05.03.2014
(73) Proprietor: Medtronic, Inc., Minneapolis, MN 55432 (US)
(72) Inventor: CERNY, Douglas S., Minneapolis, Minnesota 55401 (US); WARREN, Reginald J., Blaine, Minnesota 55449 (US); SNYDER, Van L., Ham Lake, Minnesota 55304 (US); BRIGHT, Kevin L., Maple Grove, Minnesota 55369 (US)
(74) Representative: Hughes, Andrea Michelle
(86) International application number: PCT/US2012/033465
(87) International publication number: WO 2012/148705

(56) References cited:
- WO-A1-2011/044616
- WO-A2-01/47411
- US-A1- 2007 123 946
- US-A1- 2009 063 187
- US-A1- 2009 171 414

## Description

This disclosure relates to implantable medical devices.

### BACKGROUND

A variety of medical devices are used for chronic, i.e., long-term, delivery of therapy to patients suffering from a variety of conditions, such as chronic pain, tremor, Parkinson's disease, epilepsy, urinary or fecal incontinence, sexual dysfunction, obesity, spasticity, or gastroparesis. For example, pumps or other fluid delivery devices can be used for chronic delivery of therapeutic agents, such as drugs to patients. Additionally, neurostimulators may be used to deliver electrical stimulation to one or more target tissue sites, e.g. nerve sites within a patient. These devices are intended to provide a patient with a therapeutic output to alleviate or assist with a variety of conditions. Typically, such devices are implanted in a patient and provide a therapeutic output under specified conditions on a recurring basis.

WO 01/47411 and US 2009/171414 are concerned with predicting data transfer requests.

### SUMMARY

In general, this disclosure describes techniques for executing passive background data transfers from implantable medical devices (IMDs) to external devices based on a prediction of data that will be requested by a user.

In one example, a method includes predicting a future request for data stored on an implantable medical device (IMD), detecting that a telemetry session between the IMD and an external device comprises available bandwidth, and automatically transferring data that is predicted to be requested in the future from the IMD to the external device via the available bandwidth of the telemetry session.

In another example, an implantable medical device (IMD) includes a telemetry module and a processor. The telemetry module is configured to facilitate communications between the IMD and an external device. The processor is configured to predict a future request for data stored on the IMD, detect that a telemetry session between the IMD and the external device comprises available bandwidth, and automatically transfer data that is predicted to be requested in the future from the IMD to the external device via the available bandwidth of the telemetry session.

In another example, a system includes an implantable medical device (IMD), an external device, and a processor. The external device is configured to communicate with the IMD. The processor is configured to predict a future request for data stored on the IMD, detect that a telemetry session between the IMD and the external device comprises available bandwidth, and automatically transfer data that is predicted to be requested in the future from the IMD to the external device via the available bandwidth of the telemetry session.

In another example, a system includes means for predicting a future request for data stored on an implantable medical device (IMD), means for detecting that a telemetry session between the IMD and an external device comprises available bandwidth, and means for automatically transferring data that is predicted to be requested in the future from the IMD to the external device via the available bandwidth of the telemetry session.

In another example, computer-readable storage medium stores instructions for causing a programmable processor to predict a future request for data stored on an implantable medical device (IMD), detect that a telemetry session between the IMD and the external device comprises available bandwidth, and automatically transfer data that is predicted to be requested in the future from the IMD to the external device via the available bandwidth of the telemetry session.

In another example, an external programming device includes a telemetry module and a processor. The telemetry module is configured to facilitate communications between the external programming device and an implantable medical device (IMD). The processor is configured to predict a future request for data stored on the IMD, detect that a telemetry session between the IMD and the external device comprises available bandwidth, and automatically transfer data that is predicted to be requested in the future from the IMD to the external device via the available bandwidth of the telemetry session.

The details of one or more examples disclosed herein are set forth in the accompanying drawings and the description below. Other features, objects, and advantages will be apparent from the description and drawings, and from the claims.

### BRIEF DESCRIPTION OF DRAWINGS

FIG. 1 is a conceptual diagram illustrating an example of a medical therapy system including an implantable medical device (IMD) configured to deliver therapy to a patient via a therapy delivery component connected to the IMD.
FIG. 2 is functional block diagram illustrating an example IMD in the form of an electrical stimulation device connected to a stimulation lead.
FIG. 3 is functional block diagram illustrating an example IMD in the form of a fluid delivery device connected to a catheter.
FIG. 4 is a functional block diagram illustrating an example of the external programmer shown in FIG. 1.
FIG. 5 is a flow chart illustrating an example method of predictively background transferring data between an IMD and an external device.
FIG. 6 illustrates an example navigation menu tree presented by the user interface of an external programmer.

### DETAILED DESCRIPTION

While existing implantable medical devices (IMDs), including, e.g. therapeutic agent delivery devices and electrical stimulation devices, such as implantable neurostimulators, may currently store limited amounts of data locally, such as user settings, user information, and diagnostic measurements, the trend in future IMD generations may be to increase the amount and variety of types of data stored on the IMD versus on a peripheral external device, such as an external programmer. For example, as personalization, user interaction and therapy analysis increases, so may the likelihood of the IMD storing large amounts of trended data, fluoroscopy images, patient charts, waveforms, and even the possibility for audio and video media. In current IMD implementations with limited local data storage, inductive and radio frequency (RF) telemetry techniques provide sufficient bandwidth for data transfer from the IMD without noticeable user lag times. However, as the amount of locally stored data increases and the data includes larger or more numerous packets of information, e.g., such as those associated with trend, audio and video data, IMD telemetry latency may be significantly impacted to the detriment of user experience.

In view of the foregoing trends in IMD technology, it may become increasingly advantageous to passively send data during available telemetry downtimes so that large amounts of data can already be received and ready for use upon request from an external device, e.g. a patient or physician programmer. However, employing a fixed or predetermined static priority for passively transferring data to the external device may fail to account for variations in user requests and may therefore still result in long lag times if lower priority data is requested prior to the data actually desired by the user, even if such data is passively transferred. The utility of a passive background transfer may be seen when implemented with a predictive nature.

Examples according to this disclosure include techniques for passively transferring data from an IMD to an external device, such as a programmer, by predicting the data that will be requested by a user in the near future and background transferring the predicted data from the IMD to the external device in advance of the user request. Future data request prediction algorithms employed in the following examples may employ a number of different bases upon which to make the predictions, including, e.g., predicting the future data request based on a user interface navigation state of the external device, known data request profiles for a category of users, or historical data request profiles for the current user/patient of the IMD and/or the external device communicating with the IMD. In addition to or in lieu of one or more of the foregoing prediction techniques, examples according to this disclosure may employ future data request prediction algorithms that make predictions based on the state of the IMD, including, e.g. the power level of the device, the state of one or more sensors, e.g. posture sensors or a volume sensor or other mechanism indicating the level of fluid in a reservoir of the IMD.

The following examples refer to passive background transfers of data between an IMD and one or more external devices. Passive data transfers may refer to data transfers that are initiated automatically by one or both of the IMD or the external devices without an explicit request for the data by a user. Background data transfers may refer to transfers that occur in the "background" of a communication session between the IMD and the external device(s). For example, a background data transfer may be a transfer of data between an IMD and external device during an active telemetry session that includes available bandwidth for the background transfer. In one such example, a background data transfer may be a transfer of data between an IMD and external device during an active telemetry session that is currently idle such that substantially all of the bandwidth for communication between the IMD and the external device is available. In another example, a background data transfer may be a transfer of data between an IMD and external device during an active telemetry session in which the devices are communicating but not currently using all of the available bandwidth for the session such that some or all of the remaining available bandwidth may be employed for the background transfer.

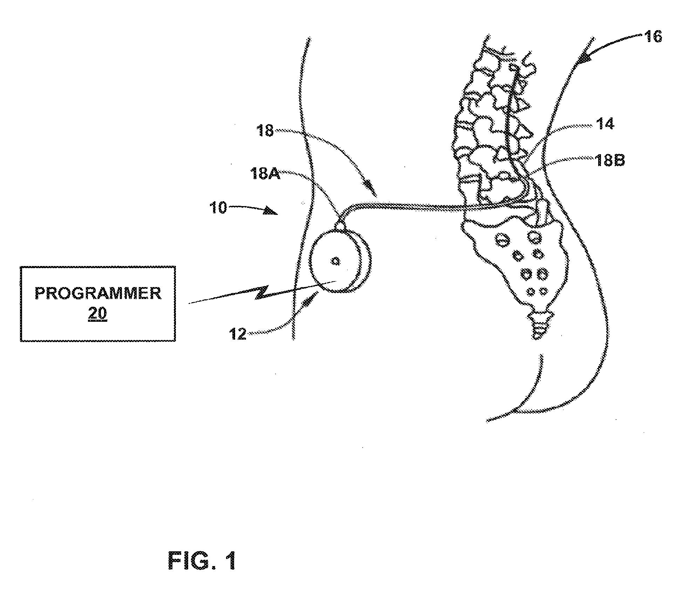

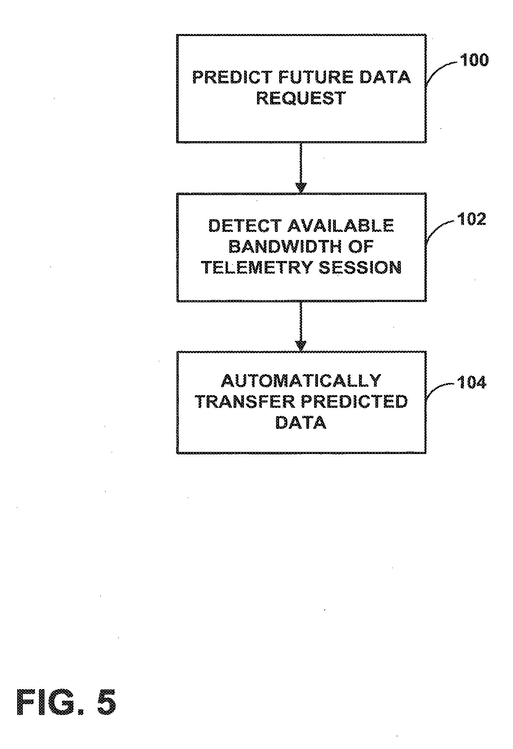

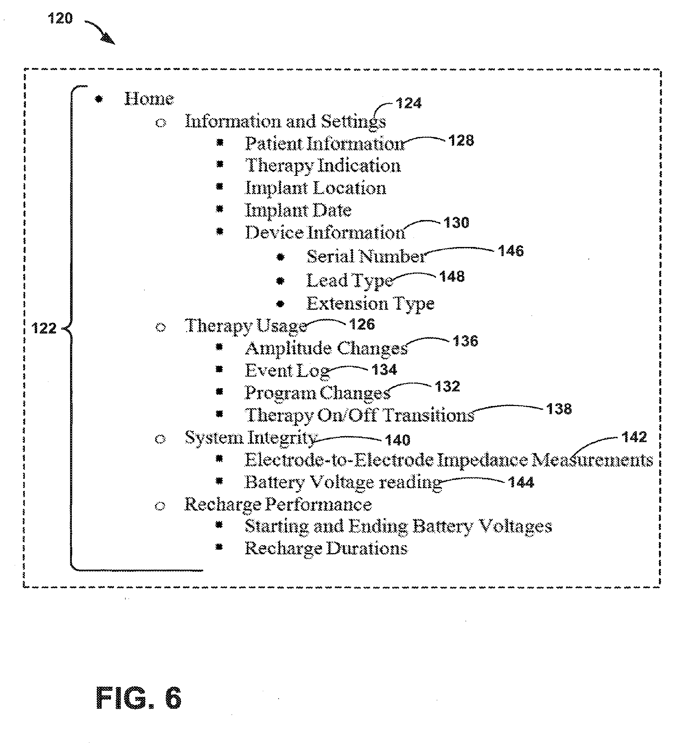

Particular techniques for predictive background data transfer from an IMD to an external device without user interaction are described in greater detail below with reference to FIGS. 5 and 6. However, an example system including a number of different possible example IMDs and an external programmer is first described with reference to FIGS. 1 - 4.

FIG. 1 is a conceptual diagram illustrating an example of a therapy system 10, which includes IMD 12, therapy delivery component 18, e.g. a catheter or electrical stimulation lead, and external programmer 20. IMD 12 is connected to therapy delivery component 18 to deliver therapy to a target site within patient 16. In one example, IMD 12 may be an implantable fluid delivery device, such as an infusion device, and therapy delivery component 18 may be a catheter that is configured to deliver at least one therapeutic agent, e.g. a pharmaceutical agent, pain relieving agent, anti-inflammatory agent, gene therapy agent, or the like, to a target site within patient 16.

In another example, IMD 12 may be an implantable neurostimulator and therapy delivery component 18 may be a lead with one or more electrodes that is configured to electrical stimulation to a target site within patient 16. External programmer 20 is configured to wirelessly communicate with IMD 12 as needed, such as to provide or retrieve therapy information or control aspects of therapy delivery (e.g., modify the therapy parameters such as rate or timing of delivery, turn IMD 12 on or off, and so forth) from IMD 12 to patient 16. In one example, communication between IMD 12 and programmer 20 may be through an external telemetry bridge device or other external perhipheral device in communication with one or both of the programmer and IMD.

IMD 12 includes an outer housing that, in some examples, is constructed of a biocompatible material that resists corrosion and degradation from bodily fluids including, e.g., titanium or biologically inert polymers. IMD 12 may be implanted within a subcutaneous pocket relatively close to the therapy delivery site. For example, in the example shown in FIG. 1, IMD 12 is implanted within an abdomen of patient 16. In other examples, IMD 12 may be implanted within other suitable sites within patient 16, which may depend, for example, on the target site within patient 16 for the delivery of, e.g., a therapeutic agent or electrical stimulation. In still other examples, IMD 12 may be external to patient 16 with a percutaneous therapy delivery component connected between IMD 12 and the target delivery site within patient 16.

In the event IMD 12 includes an electrical stimulation device, such as an implantable neurostimulator, therapy delivery component 18 may include one or more electrical stimulation leads, each of which may include one or more electrodes. In such an example, IMD 12 may be an implantable electrical stimulator that delivers neurostimulation therapy to patient 16, e.g., for relief of chronic pain or other symptoms. Again, although FIG. 1 shows an IMD, other examples may include an external stimulator, e.g., with percutaneously implanted leads.

Electrical stimulation energy, which may be constant current or constant voltage based pulses, for example, may be delivered from IMD 12 to one or more targeted locations within patient 16 via one or more electrodes (not shown) of an implantable lead connected to the IMD. In the example of FIG. 1, therapy delivery component 18 may include one or more leads carrying one or more electrodes that are placed adjacent to the target tissue near spinal cord 14 of patient 16. Electrodes of therapy delivery component 18 including a stimulation lead may transfer electrical stimulation generated by an electrical stimulation generator, e.g. a pulse generator in IMD 12 to tissue of patient 16 adjacent spinal cord 14. The electrodes employed in conjunction with IMD 12 may be electrode pads on a paddle lead, circular (e.g., ring) electrodes surrounding the body of the lead, conformable electrodes, cuff electrodes, segmented electrodes, or any other type of electrodes capable of forming unipolar, bipolar or multipolar electrode configurations for therapy. In general, ring electrodes arranged at different axial positions at the distal ends of therapy delivery component 18 will be described for purposes of illustration.

In the event therapy delivery component 18 includes one or more stimulation leads, such leads may be implanted within patient 16 directly or indirectly (e.g., via a lead extension) coupled to IMD 12. Alternatively, as mentioned above, electrical stimulation leads may be implanted and coupled to an external stimulator, e.g., through a percutaneous port. In some cases, an external stimulator is a trial or screening stimulation that is used on a temporary basis to evaluate potential efficacy to aid in consideration of chronic implantation for a patient. In additional examples, IMD 12 may be a leadless stimulator with one or more arrays of electrodes arranged on a housing of the stimulator rather than leads that extend from the housing.

The deployment of electrodes via leads 16 and 18 is described for purposes of illustration, but arrays of electrodes may be deployed in different ways. For example, a housing associated with a leadless stimulator may carry arrays of electrodes, e.g., rows and/or columns (or other patterns). Such electrodes may be arranged as surface electrodes, ring electrodes, or protrusions. As a further alternative, electrode arrays may be formed by rows and/or columns of electrodes on one or more paddle leads. In some examples, electrode arrays may include electrode segments, which may be arranged at respective positions around a periphery of a lead, e.g., arranged in the form of one or more segmented rings around a circumference of a cylindrical lead. In examples in which lead 18 is configured to sense the signals evoked by the delivery of stimulation to a dorsal root and/or peripheral nerve, lead 18 may include an array of electrodes to sense the evoked signal at a plurality of locations on the dorsal columns to provide sensing at a plurality of locations along the dorsal columns. In some examples, one or more additional electrodes may be located on or within the housing of IMD 12, e.g., to provide a common or ground electrode or a housing anode or cathode. Such a housing or case electrode may act as electrode in unipolar electrode combinations including one electrode on one of leads 16 or 18 or may be employed in a leadless configuration in which stimulation originates from the housing of IMD 12.

IMD 12 delivers electrical stimulation therapy to patient 16 via selected combinations of electrodes carried by one or both of leads 16 and 18, as well as, in some examples, an electrode on the housing of IMD 12. The target tissue for the electrical stimulation therapy may be any tissue affected by electrical stimulation energy, which may be in the form of electrical stimulation pulses or waveforms. In some examples, the target tissue includes nerves, smooth muscle, and skeletal muscle. In the example illustrated by FIG. 1, the target tissue is tissue proximate spinal cord 14, such as within an intrathecal space or epidural space of spinal cord 14, or, in some examples, adjacent nerves that branch off of spinal cord 14. Leads coupled to IMD 12 may be introduced into spinal cord 14 via any suitable region, such as the thoracic, cervical or lumbar regions. Stimulation of spinal cord 14 may, for example, prevent pain signals from traveling through spinal cord 14 and to the brain of patient 16. Patient 16 may perceive the interruption of pain signals as a reduction in pain and, therefore, efficacious therapy results.

In the event IMD 12 includes a fluid delivery device and therapy delivery component 18 includes a catheter, IMD 12 may deliver a therapeutic agent from a reservoir (not shown) to patient 16 through the catheter from proximal end 18A coupled to IMD 12 to distal end 18B located proximate to the target site. Therapy deliver component 18 connected to IMD 12 may include a unitary catheter or a plurality of catheter segments connected together to form an overall catheter length. Example therapeutic agents that may be delivered by IMD 12 via therapy delivery component 18 include, e.g., insulin, morphine, hydromorphone, bupivacaine, clonidine, other analgesics, baclofen and other muscle relaxers and antispastic agents, genetic agents, antibiotics, nutritional fluids, hormones or hormonal drugs, gene therapy drugs, anticoagulants, cardiovascular medications or chemotherapeutics.

Catheter 18 may be coupled to IMD 12 either directly or with the aid of a catheter extension (not shown in FIG. 1). In the example shown in FIG. 1, catheter 18 traverses from the implant site of IMD 12 to one or more targets proximate to spinal cord 14. Catheter 18 is positioned such that one or more fluid delivery outlets (not shown in FIG. 1) of catheter 18 are proximate to the targets within patient 16. In the example of FIG. 1, IMD 12 delivers a therapeutic agent through catheter 18 to targets proximate to spinal cord 14.

IMD 12 can be configured for intrathecal drug delivery into the intrathecal space, as well as epidural delivery into the epidural space, both of which surround spinal cord 14. In some examples, multiple catheters may be coupled to IMD 12 to target the same or different nerve or other tissue sites within patient 16, or catheter 18 may include multiple lumens to deliver multiple therapeutic agents to the patient. Therefore, although the target site shown in FIG. 1 is proximate to spinal cord 14 of patient 16, other applications of therapy system 10 include alternative target delivery sites in addition to or in lieu of the spinal cord of the patient.

In one example, IMD 12 may include a combination of an implantable fluid delivery device and electrical stimulation device. For example, IMD 12 may include an implantable infusion device and neurostimulator. In such an example, therapy delivery component 18 connected to IMD 12 may include a combination of one or more catheters and electrical stimulation leads for delivering therapeutic agents and electrical stimulation, respectively, to one or more target tissue sites within patient 16.

IMD 12 may control therapy delivered to patient 16, e.g. electrical stimulation delivered via electrodes on one or more leads of therapy delivery component 18 or a therapeutic agent delivered through a catheter, according to a program or a number of different programs executed at different times or in conjunction with different conditions, e.g. patient posture. A program defines values for one or more parameters that define an aspect of the therapy delivered by IMD 12 according to that program. The parameters for a program that controls delivery of stimulation energy by IMD 12 may include information identifying which electrodes have been selected for delivery of stimulation according to a stimulation program, the polarities of the selected electrodes, i.e., the electrode configuration for the program, and voltage or current amplitude, pulse rate, pulse shape, and pulse width of stimulation delivered by the electrodes. The parameters for a program that controls delivery of a therapeutic agent to patient 16 by IMD 12 may include information identifying the dose of therapeutic agent that is to be delivered to patient 16, as well as a schedule of one or more different therapeutic agents and/or delivery rates/doses for such agents to be delivered to the patient at different times or based on different conditions.

In some examples, therapy may be delivered by IMD 12 to patient 16 according to multiple programs, wherein multiple programs are contained within each of a plurality of groups. Each program group may support an alternative therapy selectable by patient 16, and IMD 12 may deliver therapy according to the multiple programs. IMD 12 may rotate through the multiple programs of the group when delivering, e.g. stimulation such that numerous conditions of patient 16 are treated. As an illustration, in some cases, stimulation pulses formulated according to parameters defined by different programs may be delivered on a time-interleaved basis. For example, a group may include a program directed to leg pain, a program directed to lower back pain, and a program directed to abdomen pain. Alternatively, multiple programs may contribute to an overall therapeutic effect with respect to a particular type or location of pain. In this manner, IMD 12 may treat different symptoms substantially simultaneously or contribute to relief of the same symptom.

A user, such as a clinician or patient 16, may interact with a user interface of external programmer 20 to program IMD 12. Programming of IMD 12 may refer generally to the generation and transfer of commands, programs, or other information to control the operation of IMD 12. For example, external programmer 20 may transmit programs, parameter adjustments, program selections, group selections, or other information to control the operation of IMD 12, e.g., by wireless telemetry. As one example, external programmer 20 may transmit particular electrode combinations for stimulation delivered by IMD 12 or particular therapeutic agent doses/delivery rates for an agent delivered to patient 16 by IMD 12. As another example, a user may select programs or program groups.

In some cases, external programmer 20 may be characterized as a physician or clinician programmer if it is primarily intended for use by a physician or clinician. In other cases, external programmer 20 may be characterized as a patient programmer if it is primarily intended for use by a patient. In another example, external programmer 20 may function as both a physician and patient programmer, e.g. based on user credentials input by the user to access functions on the programmer. A patient programmer is generally accessible to patient 16 and, in many cases, may be a portable device that may accompany the patient throughout the patient's daily routine. In general, a physician or clinician programmer may support selection and generation of programs by a clinician for use by IMD 12, whereas a patient programmer may support adjustment and selection of such programs by a patient during ordinary use.

As described in greater detail below, in examples according to this disclosure, therapy system 10 of FIG. 1 is configured to passively transfer data from IMD 12 to external programmer 20, or another external device by predicting the particular data that may be requested by a user in the near future and background transferring the predicted data from the IMD to the external device in advance of the user request. For example, system 10 may include a data transfer module that monitors the state of telemetry communications between IMD 12 and programmer 20, or another external device communicatively connected to IMD 12, and background transfers data that the module predicts will be requested by a user from IMD 12 to programmer 20 during the active telemetry session and in advance of the user requesting the data. The background transfer may include transfer of data between IMD 12 and programmer 20 during an active telemetry session that includes available bandwidth for the background transfer. For example, a background data transfer may be a transfer of data between IMD 12 and programmer 20 during an active telemetry session that is currently idle. The data transfer module may be implemented in hardware, software, or combinations thereof and may be executed by or included in, in whole or in part, one or both of IMD 12 and programmer 20, or another external device communicatively connected to IMD 12. The data transfer module may include algorithms employing a number of different models upon which to make predictions, including, e.g., predicting a future data request based on a user interface navigation state of an external device, known data request profiles for a category of users, or historical data request profiles for the current user/patient of IMD 12 and/or the external device communicating with the IMD, e.g. external programmer 20.

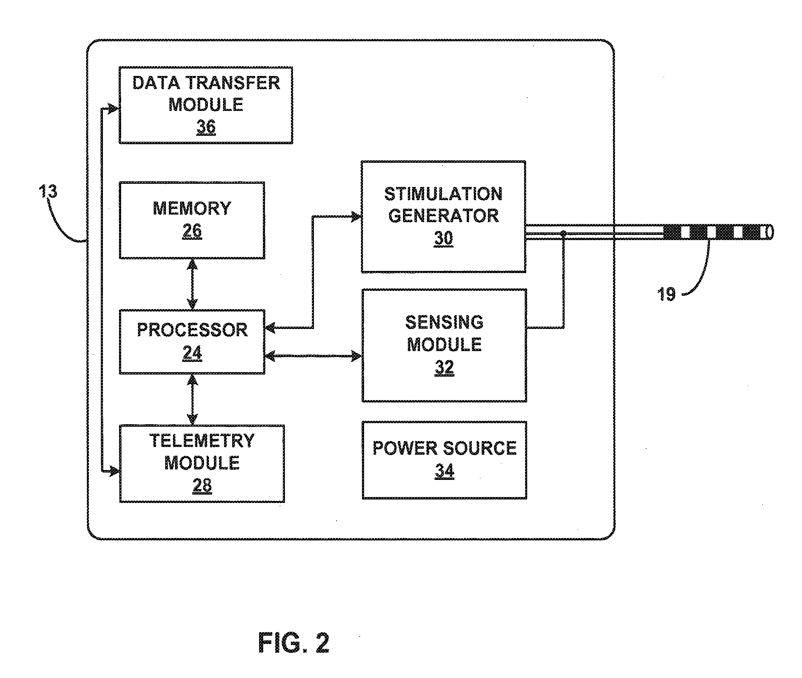

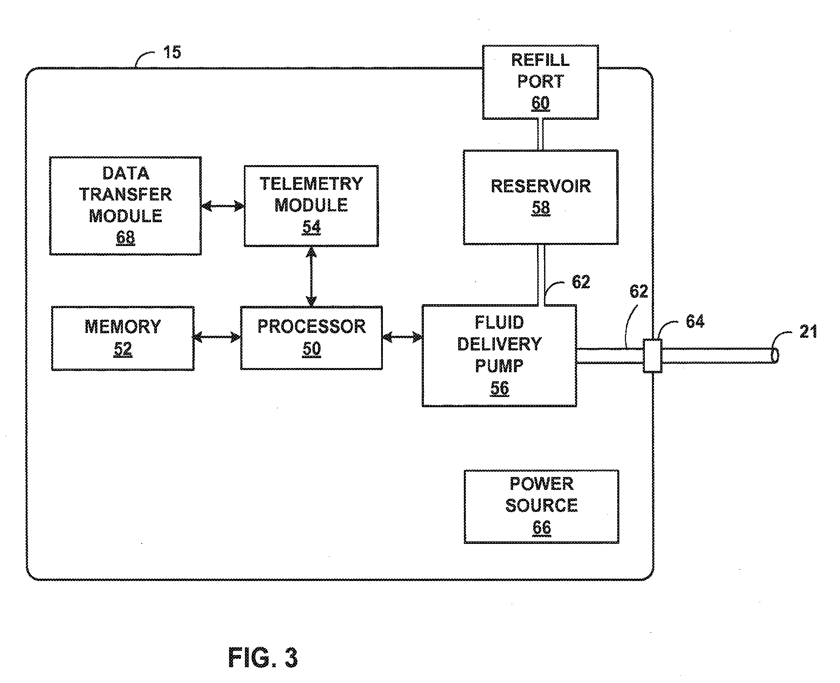

FIGS. 2 and 3 are functional block diagrams illustrating various components of example IMDs that may be employed in examples according to this disclosure. FIG. 2 is a functional block diagram illustrating various components of an example IMD that is configured as an implantable electrical stimulation device, e.g. a neurostimulator for a spinal cord stimulation (SCS) system. FIG. 3 is a functional block diagram illustrating various components of an example IMD that is configured as an implantable infusion device, e.g. a drug pump for intrathecal deliver of one or more therapeutic agents to a patient. The example IMDs of FIGS. 2 and 3 include a number of commonly configured and functioning components, e.g. processor(s), memory, telemetry modules, power sources, etc. Additionally, each includes a data transfer module that is configured in accordance with examples according to this disclosure to passively transfer data from the IMD to an external device by predicting the particular data that may be requested by a user in the near future and background transferring the predicted data from the IMD to the external device in advance of the user request.

FIG. 2 is a functional block diagram illustrating various components of IMD 13. In the example of FIG. 2, IMD 13 includes processor 24, memory 26, telemetry module 28, stimulation generator 30, sensing module 32, power source 34, and data transfer module 36. IMD 13 may be an implantable electrical stimulator, e.g. a neurostimulator configured to deliver stimulation therapy to patient 16, e.g. configured to deliver stimulation to spinal cord 14 of the patient. For example, processor 24 may control stimulation generator 30, e.g. according to one or more programs to deliver electrical stimulation via electrodes on lead 18 to spinal cord 14 of patient 16.

In one example, processor 24 controls stimulation generator 30 to deliver electrical stimulation via electrode combinations formed by electrodes in one or more electrode arrays. For example, stimulation generator 30 may deliver electrical stimulation therapy via electrodes on lead 18, e.g., as stimulation pulses or continuous waveforms. Components described as processors within IMD 13, external programmer 20 or any other device described in this disclosure may each comprise one or more processors, such as one or more microprocessors, digital signal processors (DSPs), application specific integrated circuits (ASICs), field programmable gate arrays (FPGAs), programmable logic circuitry, or the like, either alone or in any suitable combination. The functions attributed to processors described herein may be embodied as software, firmware, hardware, or any combination thereof.

Stimulation generator 30 may include stimulation generation circuitry to generate stimulation pulses or waveforms and switching circuitry to switch the stimulation across different electrode combinations, e.g., in response to control by processor 24. In particular, processor 24 may control the switching circuitry on a selective basis to cause stimulation generator 30 to deliver electrical stimulation to selected electrode combinations and to shift the electrical stimulation to different electrode combinations in a first direction or a second direction when the therapy must be delivered to a different location within patient 16. In other examples, stimulation generator 30 may include multiple current sources to drive more than one electrode combination at one time. In this case, stimulation generator 30 may decrease current to the first electrode combination and simultaneously increase current to the second electrode combination to shift the stimulation therapy. In another example, IMD 13 may include multiple stimulation generators, each of which may be capable of driving an electrode combination such that multiple combinations may be driven simultaneously by the multiple generators.

An electrode configuration, e.g., electrode combination and associated electrode polarities, may be represented by a data stored in a memory location, e.g., in memory 26, of IMD 13. Processor 24 may access the memory location to determine the electrode combination and control stimulation generator 30 to deliver electrical stimulation via the indicated electrode combination. To adjust electrode combinations, amplitudes, pulse rates, or pulse widths, processor 24 may command stimulation generator 30 to make the appropriate changes to therapy according to instructions within memory 26 and rewrite the memory location to indicate the changed therapy. In other examples, rather than rewriting a single memory location, processor 24 may make use of two or more memory locations.

When activating stimulation, processor 24 may access not only the memory location specifying the electrode combination but also other memory locations specifying various stimulation parameters such as voltage or current amplitude, pulse width and pulse rate. Stimulation generator 30, e.g., under control of processor 24, then makes use of the electrode combination and parameters in formulating and delivering the electrical stimulation to patient 16.

Processor 24 accesses stimulation parameters in memory 26, e.g., as programs and groups of programs. Upon selection of a particular program group, processor 24 may control stimulation generator 30 to generate and deliver stimulation according to the programs in the groups, e.g., simultaneously or on a time-interleaved basis. A group may include a single program or multiple programs. As mentioned previously, each program may specify a set of stimulation parameters, such as amplitude, pulse width and pulse rate. In addition, each program may specify a particular electrode combination for delivery of stimulation. Again, the electrode combination may specify particular electrodes in a single array or multiple arrays, e.g., on a single lead or among multiple leads.

Memory 26 may include any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media. Memory 26 may store instructions for execution by processor 24, stimulation therapy data, predictive data request algorithms, historical or population data regarding past user data requests, and any other information regarding therapy delivered by IMD 13 or patient 16. Therapy information may be recorded for long-term storage and retrieval by a user, and the therapy information may include any data created by or stored in IMD 13. Memory 26 may include separate memories for storing instructions, sensed signal information, program histories, and any other data that may benefit from separate physical memory modules.

Memory 26 may be considered, in some examples, a non-transitory computer-readable storage medium comprising instructions that cause one or more processors, such as, e.g., processor 24, to implement one or more of the example techniques described in this disclosure. The term "non-transitory" may indicate that the storage medium is not embodied in a carrier wave or a propagated signal. However, the term "non-transitory" should not be interpreted to mean that memory 26 is non-movable. As one example, memory 26 may be removed from IMD 13, and moved to another device. In certain examples, a non-transitory storage medium may store data that can, over time, change (e.g., in RAM).

Sensing module 32 may be configured to monitor one or more signals from one or more electrodes on lead 18 in order to monitor electrical activity at one more locations in patient 16, e.g., via electrogram (EGM) signals. For example, sensing module 32 may be configured to monitor one or more electrical signals from electrode(s) on lead 18 at one or more locations along spinal cord 14 of patient 16. Sensing module 32 may also include a switch module to select which of the available electrodes, or which pairs or combinations of electrodes, are used to sense the electrical activity at locations within patient 16. In some examples, sensing module 32 includes one or more sensing channels, each of which may comprise an amplifier. In response to signals from processor 24, the switch module within sensing module 32 may couple the outputs from the selected electrodes to one of the sensing channels. Sensed signal data from sensing module 32 may be stored in memory 26 for later analysis by processor 24, review by a clinician, used to adjust therapy parameter (e.g., electrode combination), transmission to programmer 20 or other external device, or some combination thereof.

IMD 13 wirelessly communicates with external programmer 20, e.g., a patient programmer or a clinician programmer, or another device by radio frequency (RF) communication or proximal inductive interaction of IMD 13 with external programmer 20. Telemetry module 28 in IMD 13, as well as telemetry modules in other devices described in this disclosure, such as programmer 20, can be configured to use RF communication techniques to wirelessly send and receive information to and from other devices respectively according to, e.g., the 802.11 or Bluetooth specification sets, or other standard or proprietary telemetry protocols. In addition, telemetry module 30 may communicate with programmer 20 via proximal inductive interaction between IMD 15 and the external programmer. Telemetry module 28 may send information to and receive information from external programmer 20 on a continuous basis, at periodic intervals, at non-periodic intervals, or upon request from the stimulator or programmer. To support RF communication, telemetry module 28 may include appropriate electronic components, such as one or more antennas, amplifiers, filters, mixers, encoders, decoders, and the like.

Power source 34 delivers operating power to the components of IMD 13. Power source 34 may include a small rechargeable or non-rechargeable battery and a power generation circuit to produce the operating power. Recharging may be accomplished through proximal inductive interaction between an external charger and an inductive charging coil within IMD 13. In some examples, power requirements may be small enough to allow IMD 13 to utilize patient motion and implement a kinetic energy-scavenging device to trickle charge a rechargeable battery. In other examples, traditional batteries may be used for a limited period of time. As a further alternative, an external inductive power supply could transcutaneously power IMD 13 when needed or desired.

IMD 13 of FIG. 2 also includes data transfer module 36. Data transfer module 36, alone or in conjunction with other devices, e.g. an external programmer, may be configured to passively transfer data from IMD 13 to external programmer 20, or another external device by predicting the particular data that may be requested by a user in the near future and automatically transferring the predicted data from the IMD to the external device in advance of the user request. Data transfer module 36 may be configured to execute the automatic predictive data transfer transparently to the user in the background, e.g. while an active telemetry session between telemetry module 28 of IMD 13 and the external device is idle.

In one example, data transfer module 36 may monitor the state of telemetry communications between telemetry module 28 of IMD 13 and a telemetry module of programmer 20, or another external device communicatively connected to IMD 13 for available bandwidth. In one example, before or after detecting an active telemetry session between IMD 13 and the external device is idle, data transfer module 36 may predict a future request by a user for data stored on the IMD and automatically transfer the predicted data from the IMD to the external device while the telemetry session is idle and in advance of the user actually requesting the data. Data transfer module 36 may include algorithms employing a number of different models upon which to execute data request predictions, including, e.g., predicting a future data request based on a user interface navigation state of an external device, known data request profiles for a category of users, or historical data request profiles for the current user/patient of IMD 13 and/or the external device communicating with the IMD, e.g. external programmer 20. Example methods by which data transfer module 36 of IMD 13 may execute data request predictions are described in more detail below with reference to FIGS. 5 and 6.

Data transfer module 36 may be implemented, at least in part, in hardware, software, firmware or any combination thereof. For example, various functions of data transfer module 36 may be implemented within one or more processors, including one or more microprocessors, digital signal processors (DSPs), application specific integrated circuits (ASICs), field programmable gate arrays (FPGAs), or any other equivalent integrated or discrete logic circuitry, as well as any combinations of such components. For example, all or some of the various functions of data transfer module 36 may be implemented within processor 24 of IMD 13. The term "processor" or "processing circuitry" may generally refer to any of the foregoing logic circuitry, alone or in combination with other logic circuitry, or any other equivalent circuitry. Some or all of the information necessary for the operation of data transfer module 36, e.g. predictive data request algorithms, may also be embodied or encoded in a computer-readable medium, such as a computer-readable storage medium, containing instructions, e.g. memory 26 of IMD 13. Instructions embedded or encoded in a computer-readable storage medium may cause a programmable processor, e.g., processor 24 or other processor, to perform the functions associated with data transfer module 36, e.g., when the instructions are executed.

FIG. 3 is a functional block diagram illustrating components of an example of IMD 15, which includes processor 50, memory 52, telemetry module 54, fluid delivery pump 56, reservoir 58, refill port 60, internal tubing 62, catheter access port 64, and power source 66. Processor 50 is communicatively connected to memory 52, telemetry module 54, and fluid delivery pump 56. Fluid delivery pump 56 is connected to reservoir 58 via internal tubing 62. Reservoir 58 is connected to refill port 60. Catheter access port 64 is connected to internal tubing 62 and catheter 21. IMD 15 may be an implantable fluid delivery device, e.g., an implantable infusion pump configured to deliver a therapeutic agent to patient 16, e.g. configured to the agent intrathecally to spinal cord 14 of the patient. For example, processor 50 may control pump 56, e.g., according to one or more programs stored in memory 52 to deliver a therapeutic agent from reservoir 58 through catheter 21 to spinal cord 14.

IMD 15 also includes power source 44, which is configured to deliver operating power to various components of the IMD. In some examples, IMD 15 may include a number of reservoirs for storing more than one type of therapeutic agent. In some examples, IMD 15 may include a single long tube that contains the therapeutic agent in place of a reservoir. However, for ease of description, an IMD 15 including a single reservoir 58 is primarily described with reference to the disclosed examples.

During operation of IMD 15, processor 50 controls fluid delivery pump 56 with the aid of instructions associated with program information that is stored in memory 52 to deliver a therapeutic agent to patient 16 via catheter 21. Instructions executed by processor 50 may, for example, define therapy programs that specify the dose of therapeutic agent that is delivered to a target tissue site within patient 16 from reservoir 58 via catheter 21. The programs may further specify a schedule of different therapeutic agent rates and/or other parameters by which IMD 15 delivers therapy to patient 16.

In general, a therapy program stored on memory 52 and executed by processor 50 defines one or more therapeutic agent doses to be delivered from reservoir 58 to patient 16 through catheter 21 by IMD 15. A dose of therapeutic agent generally refers to a total amount of therapeutic agent, e.g., measured in milligrams or other volumetric units, delivered over a total amount of time, e.g., per day or twenty-four hour period. The amount of therapeutic agent in a dose may convey to a caregiver an indication of the probable efficacy of the fluid and the possibility of side effects.

In general, a sufficient amount of the fluid should be administered in order to have a desired therapeutic effect, such as pain relief. However, the amount of the therapeutic agent delivered to the patient should be limited to a maximum amount, such as a maximum daily amount, in order not to avoid potential side effects. Therapy program parameters specified by a user, e.g., via programmer 20 may include fluid volume per dose, dose time period, maximum dose for a given time interval e.g., daily. In some examples, dosage may also prescribe particular concentrations of active ingredients in the therapeutic agent delivered by IMD 15 to patient 16.

The manner in which a dose of therapeutic agent is delivered to patient 16 by IMD 15 may also be defined in the therapy program. For example, processor 50 of IMD 15 may be programmed to deliver a dose of therapeutic agent according to a schedule that defines different rates at which the fluid is to be delivered at different times during the dose period, e.g. a twenty-four hour period. The therapeutic agent rate refers to the amount, e.g. in volume, of therapeutic agent delivered over a unit period of time, which may change over the course of the day as IMD 15 delivers the dose of fluid to patient 16. A therapy program may include other parameters, including, e.g., definitions of priming and patient boluses, as well as time intervals between successive patient boluses, sometimes referred to as lock-out intervals.

Therapy programs may be a part of a program group, where the group includes a number of therapy programs. Memory 52 of IMD 15 may store one or more therapy programs, as well as instructions defining the extent to which patient 16 may adjust therapy parameters, switch between therapy programs, or undertake other therapy adjustments. Patient 16 or a clinician may select and/or generate additional therapy programs for use by IMD 15, e.g., via external programmer 20 at any time during therapy or as designated by the clinician.

Upon instruction from processor 50, fluid delivery pump 56 may draw fluid from reservoir 58 and pump the fluid through internal tubing 62 to catheter 21 through which the fluid is delivered to patient 16 to effect one or more of the treatments, e.g., in accordance with a program stored on memory 52. Internal tubing 62 may be a segment of tubing or a series of cavities within IMD 15 that run from reservoir 58, around or through fluid delivery pump 56 to catheter access port 64 and catheter 21.

Fluid delivery pump 56 can be any mechanism that delivers a therapeutic agent in some metered or other desired flow dosage to the therapy site within patient 16 from reservoir 58 via implanted catheter 21. In one example, fluid delivery pump 56 is a squeeze pump that squeezes internal tubing 62 in a controlled manner, e.g., such as a peristaltic pump, to progressively move fluid from reservoir 58 to the distal end of catheter 21 and then into patient 16 according to parameters specified by the therapy program stored on memory 52 and executed by processor 50.

In various examples, fluid delivery pump 56 may be an axial pump, a centrifugal pump, a pusher plate pump, a piston-driven pump, or other means for moving fluid through internal tubing 62 and catheter 21. In one example, fluid delivery pump 56 is an electromechanical pump that delivers fluid by the application of pressure generated by a piston that moves in the presence of a varying magnetic field and that is configured to draw fluid from reservoir 58 and pump the fluid through internal tubing 62 and catheter 21 to patient 16.

Periodically, fluid may need to be supplied percutaneously to reservoir 58 because all of a therapeutic agent has been or will be delivered to patient 16, or because a clinician wishes to replace an existing fluid with a different fluid or similar fluid with different concentrations of therapeutic ingredients. Refill port 60 can therefore comprise a self-sealing membrane to prevent loss of therapeutic agent delivered to reservoir 58 via refill port 60. For example, after a percutaneous delivery system, e.g., a hypodermic needle, penetrates the membrane of refill port 60, the membrane may seal shut when the needle is removed from refill port 60.

In general, memory 52 stores program instructions and related data that, when executed by processor 50, cause IMD 15 and processor 50 to perform the functions attributed to them in this disclosure. For example, memory 52 of IMD 15 may store instructions for execution by processor 50 and/or data transfer module 68 including, e.g., therapy programs, data transfer prediction algorithms, and any other information regarding therapy delivered to patient 16 and/or the operation of IMD 15. Memory 52 may include separate memories for storing instructions, patient information, therapy parameters, therapy adjustment information, program histories, and other categories of information such as any other data that may benefit from separate physical memory modules. Therapy adjustment information may include information relating to timing, frequency, rates and amounts of patient boluses or other permitted patient modifications to therapy.

At various times during the operation of IMD 15 to treat patient 16, communication to and from IMD 15 may be necessary to, e.g., change therapy programs, adjust parameters within one or more programs, configure or adjust a particular bolus, or to otherwise download information to or from IMD 15. Processor 50 controls telemetry module 54 to wirelessly communicate between IMD 15 and other devices including, e.g. programmer 20. Telemetry module 54 of IMD 15 may wirelessly communicate with external programmer 20, e.g., a patient programmer or a clinician programmer, or another device by radio frequency (RF) communication or proximal inductive interaction of IMD 13 with external programmer 20. Telemetry module 54 of IMD 15 may send information to and receive information from external programmer 20 on a continuous basis, at periodic intervals, at non-periodic intervals, or upon request from the stimulator or programmer. To support RF communication, telemetry module 54 may include appropriate electronic components, such as one or more antennas, amplifiers, filters, mixers, encoders, decoders, and the like.

Power source 66 delivers operating power to various components of IMD 15. Power source 66 may include a small rechargeable or non-rechargeable battery and a power generation circuit to produce the operating power. In the case of a rechargeable battery, recharging may be accomplished through proximal inductive interaction between an external charger and an inductive charging coil within IMD 15. In some examples, power requirements may be small enough to allow IMD 15 to utilize patient motion and implement a kinetic energy-scavenging device to trickle charge a rechargeable battery. In other examples, traditional batteries may be used for a limited period of time. As another alternative, an external inductive power supply may transcutaneously power IMD 15 as needed or desired.

IMD 15 of FIG. 3 also includes data transfer module 68. Data transfer module 68, alone or in conjunction with other devices, e.g., an external programmer, may be configured to passively transfer data from IMD 15 to external programmer 20, or another external device by predicting the particular data that may be requested by a user in the near future and automatically transferring the predicted data from the IMD to the external device in advance of the user request. Data transfer module 68 may be configured to execute the automatic predictive data transfer transparently to the user in the background, e.g. while an active telemetry session between telemetry module 54 of IMD 15 and the external device is idle.

In one example, data transfer module 68 may monitor the state of telemetry communications between telemetry module 54 of IMD 15 and a telemetry module of programmer 20, or another external device communicatively connected to IMD 15 for available bandwidth. In one example, before or after detecting an active telemetry session between IMD 15 and the external device is idle, data transfer module 68 may predict a future request by a user for data stored on the IMD and automatically transfer the predicted data from the IMD to the external device while the telemetry session is idle and in advance of the user actually requesting the data. Data transfer module 68 may include algorithms employing a number of different models upon which to execute data request predictions, including, e.g., predicting a future data request based on a user interface navigation state of an external device, known data request profiles for a category of users, or historical data request profiles for the current user/patient of IMD 13 and/or the external device communicating with the IMD, e.g. external programmer 20. Example methods by which data transfer module 68 of IMD 15 may execute data request predictions are described in more detail below with reference to FIGS. 5 and 6.

Data transfer module 68 may be implemented and function in a similar manner as described above with reference to data transfer module 36 of example IMD 13. As such, data transfer module 68 may be implemented to function in accordance with examples according to this disclosure, at least in part, in hardware, software, firmware or any combination thereof, as described in more detail above with reference to data transfer module 36 of example IMD 13.

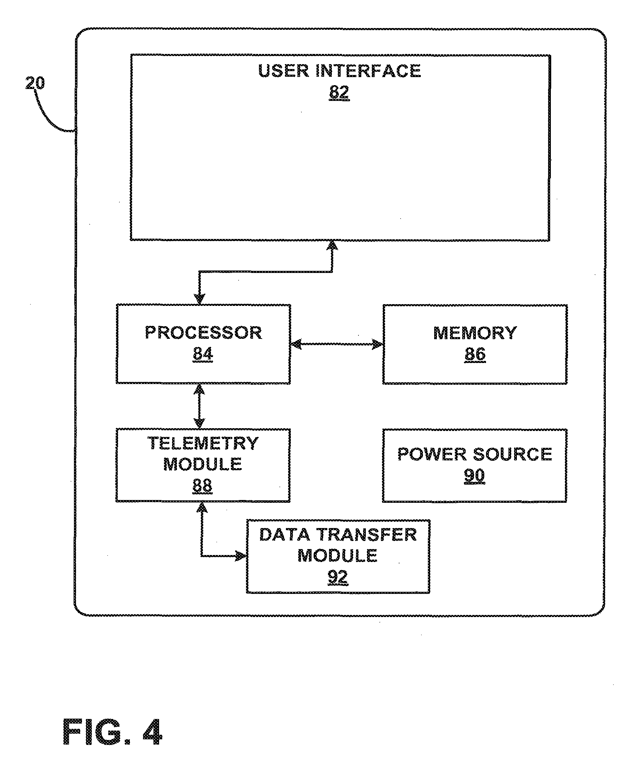

FIG. 4 is a functional block diagram illustrating an example of various components of external programmer 20, which may be configured to communicate with and program operation of an IMD, e.g. IMD 13 of FIG. 2 or IMD 15 of FIG. 3. As shown in FIG. 4, example external programmer 20 includes user interface 82, processor 84, memory 86, telemetry module 88, power source 90, and data transfer module 92. A clinician or patient 16 interacts with user interface 82 in order to manually change the parameters of a therapy program, change therapy programs within a group of programs, view therapy information, view historical or establish new therapy programs, or otherwise communicate with an IMD, e.g. IMD 13 or 15, or view or edit programming information. Processor 84 controls user interface 82, retrieves data from memory 86 and stores data within memory 86. Processor 84 also controls the transmission of data through telemetry module 88 to the IMD, e.g. to telemetry module 28 of IMD 13 or telemetry module 54 of IMD 15. The transmitted data may include therapy program information specifying various therapeutic agent delivery parameters. Memory 86 may store, e.g., operational instructions for processor 84 and data related to therapy for patient 16 and operation of data transfer module 92.

Programmer 20 may be a hand-held computing device that includes user interface 82 that can be used to provide input to programmer 20. For example, programmer 20 may include a display screen that presents information to the user and a keypad, buttons, a peripheral pointing device, touch screen, voice recognition, or another input mechanism that allows the user to navigate though the user interface of programmer 20 and provide input. In other examples, rather than being a handheld computing device or a dedicated computing device, programmer 20 may be a larger workstation or a separate application within another multifunction device.

When programmer 20 is configured for use by a clinician, user interface 82 may be used to transmit initial programming information to an IMD including hardware information for a medical system including the IMD and programmer, e.g. the type of therapy delivery component 18 in system 10 of FIG. 1, the position of therapy delivery component 18 within patient 16, and software information related to therapy delivery and operation of the IMD, e.g. therapy parameters of therapy programs stored within the IMD or within programmer 20, and any other information the clinician desires to program into or transfer to the IMD. The clinician may use programmer 20 during a programming session to define one or more therapy programs by which the IMD delivers therapy to patient 16, in which case patient 16 may provide feedback to the clinician during the programming session as to efficacy of a program being evaluated or desired modifications to the program. Programmer 20 may assist the clinician in the creation/identification of therapy programs by providing a methodical system of identifying potentially beneficial therapy parameters.

Programmer 20 may also be configured for use by patient 16. When configured as a patient programmer, programmer 20 may have limited functionality in order to prevent patient 16 from altering critical functions or applications that may be detrimental to patient 16. In this manner, programmer 20 may only allow patient 16 to adjust certain therapy parameters or set an available range for a particular therapy parameter. For example, in the event programmer 20 is employed in conjunction with example IMD 15 of FIG. 3, a patient programmer may permit the patient to control IMD 15 to deliver a supplemental, patient bolus, if permitted by the applicable therapy program administered by the IMD, e.g., if delivery of a patient bolus would not violate a lockout interval or maximum dosage limit. Programmer 20 may also provide an indication to patient 16 when therapy is being delivered or when the power source within programmer 20 or the IMD need to be replaced or recharged.

Telemetry module 88 allows the transfer of data to and from programmer 20 and an IMD, e.g. IMD 13 or IMD 15, as well as other devices, e.g. according to the RF communication techniques described above with reference to FIGS. 2 and 3. Telemetry module 88 may communicate automatically with the IMD at a scheduled time or when the telemetry module detects the proximity of the IMD. Alternatively, telemetry module 88 may communicate with the IMD when signaled by a user through user interface 82. To support RF communication, telemetry module 88 may include appropriate electronic components, such as amplifiers, filters, mixers, encoders, decoders, and the like. Programmer 20 may also communicate with another programmer or computing device via a wired or wireless connection using any of a variety of communication techniques, and/or via exchange of removable media, including, e.g., magnetic or optical disks, or memory cards or sticks including, e.g., non-volatile memory. Further, programmer 20 may communicate with another device via, e.g., a local area network (LAN), wide area network (WAN), public switched telephone network (PSTN), or cellular telephone network, or any other terrestrial or satellite network appropriate for use with programmer 20.

Power source 90 may be a rechargeable battery, such as a lithium ion or nickel metal hydride battery. Other rechargeable or conventional primary cell batteries may also be used. In some cases, external programmer 20 may be used when coupled to an alternating current (AC) outlet, i.e., AC line power, either directly or via an AC/DC adapter.

In some examples, external programmer 20 may be configured to recharge an IMD in addition to programming the device. Alternatively, a recharging device may be capable of communication with the IMD. Then, the recharging device may be able to transfer programming information, data, or any other information described herein to the IMD. In this manner, the recharging device may be able to act as an intermediary communication device between external programmer 20 and the IMD with which the programmer is being employed. In such cases including an external recharging device capable of communication with an IMD, the recharging device may be employed to execute passive background data transfers from the IMD to the recharging device based on a prediction of data that will be requested by a user in accordance with this disclosure. For example, there may be circumstances including a relatively long recharge cycle between an IMD and an external recharge device that may include periods during which the communicatin session is idle or otherwise includes available bandwidth for predictive background transfers between the IMD and the recharging device.

Referring again to FIG. 4, external programmer 20 also includes data transfer module 92. Data transfer module 92, alone or in conjunction with other devices, e.g. an external programmer, may be configured to passively transfer data from IMD 15 to external programmer 20, or another external device by predicting the particular data that may be requested by a user in the near future and automatically transferring the predicted data from the IMD to the external device in advance of the user request. Data transfer module 92 may be configured and function in a manner similar to data transfer module 36 of IMD 13 described above with reference to FIG. 2 or data transfer module 68 of IMD 15 described above with reference to FIG. 3.

In one example, data transfer module 92 of external programmer may act alone, without the assistance of a data transfer module on board the IMD with which the programmer is communicating. For example, data transfer module 92 may monitor the state of telemetry communications between a telemetry module of an IMD and telemetry module 88 of programmer 20 for available bandwidth. In one example, before or after detecting an active telemetry session between the IMD and programmer 20 is idle, data transfer module 92 may predict a future request by a user for data stored on the IMD and automatically transfer the predicted data from the IMD to the external programmer while the telemetry session is idle and in advance of the user actually requesting the data. Data transfer module 92 may include algorithms employing a number of different models upon which to execute data request predictions, including, e.g., predicting a future data request based on a user interface navigation state of an external device, known data request profiles for a category of users, or historical data request profiles for the current user/patient of the IMD and/or external programmer 20. Example methods by which data transfer module 92 of external programmer 20 may execute data request predictions are described in more detail below with reference to FIGS. 5 and 6.

In another example, data transfer module 92 may act in conjunction with another data transfer module on board an IMD or other components of the IMD to predictively transfer data from the IMD to external programmer 20 in the background, e.g. during an idle telemetry session and without interaction from the user. For example, data transfer module 92 of programmer 20 may act in conjunction with data transfer module 36 of IMD 13 or data transfer module 68 of IMD 15 to predictively background transfer data from the IMD to the external programmer. In one example, data transfer module 92 of programmer 20 may monitor the state of telemetry communications between telemetry module 54 of IMD 15 and telemetry module 88 of programmer 20. Before or after detecting an active telemetry session between IMD 15 and the external device is idle, data transfer module 92 of programmer 20 may instruct data transfer module 68 of IMD 15 to predict a future request by a user for data stored on the IMD and automatically transfer the predicted data from the IMD to the programmer while the telemetry session is idle and in advance of the user actually requesting the data.

FIG. 5 is a flow chart illustrating an example method of predictively background transferring data between an IMD and an external device, e.g. an external user or clinician programmer. The method of FIG. 5 includes predicting a future request for data stored on the IMD (100), detecting that a telemetry session between an IMD and an external device includes available bandwidth (102), and automatically transferring data that is predicted to be requested in the future from the IMD to the external device via the available bandwidth of the telemetry session(104). The functions of the method of FIG. 5 are described as executed by programmer 20, and in particular, data transfer module 92 of programmer 20. However, in other examples, one or more of these functions may be carried out by and/or in conjunction with other devices including, e.g., data transfer module 36 of example IMD 13 of FIG. 2 or transfer module 68 of example IMD 15 of FIG. 3. Additionally, although programmer 20 may be employed with IMD 13 of FIG. 2 or IMD 15 of FIG. 3, or various other IMDs of different configurations, for simplicity, the following examples include programmer 20 communicating and otherwise interacting with example IMD 13 described above with reference to FIG. 2.

The example method of FIG. 5 includes predicting a future request for data stored on the IMD (100). As noted several times above, data transfer module 92 may include algorithms employing a number of different models upon which to execute data request predictions according to this disclosure, including, e.g., predicting a future data request based on a user interface navigation state of an external device, known data request profiles for a category of users, or historical data request profiles for the current user/patient of IMD 13 and/or external programmer 20. In another example, data transfer module 92 may include algorithms that make predictions based on the state of IMD 13, including, e.g. the power level of power source 34, the state of one or more sensors, e.g. posture sensors or a volume sensor or other mechanism indicating the level of fluid in reservoir 58 of fluid deliver IMD 15 of FIG. 3.

In one example, data transfer module 92 may be configured to predict a future request for data stored on IMD 13 based on the state of user interface 82 of external programmer 20. In the course of making such data request predictions, data transfer module 92 may also be configured to detect various states of user interface 82 of external programmer 20. Data transfer module 92 may be configured to actively detect states of user interface 82, e.g. by querying the user interface for state information or requesting that processor 84 of programmer 20 execute such a query. In another example, data transfer module 92 my be configured to infer the state of user interface 82 from data or other information being exchanged between programmer 20 and IMD 15 during an active telemetry session. For example, data transfer module 92 may monitor the telemetry session between programmer 20 and IMD 15 and infer the state of user interface 82 from the particular data that was most recently exchanged between the two devices.

In one example, data transfer module 92 may be configured to detect an active branch in a navigation menu tree presented to the clinician on user interface 82 of external programmer 20. FIG. 6 illustrates example navigation menu tree 120 that may be presented by user interface 82 of programmer 20 in conjunction with communications with IMD 13. Navigation menu tree 120 is presented in FIG. 6 to illustrate the content and hierarchical structure of the tree, but not necessarily the manner in which user interface 82 will present the tree to the clinician using programmer 20. For example, user interface 82 may present navigation menu tree 120 to the clinician graphically on a display of the programmer in a horizontal orientation, instead of the vertical orientation illustrated in FIG. 6. At any rate, navigation menu tree 120 includes a number of branches 122, including, e.g. a number of first-level branches, e.g., Information and Settings branch 124 and Therapy Usage 126, as well as sub-branches, e.g., Patient information branch 128. First-level branches, as used in this disclosure, does not necessarily refer to top-level branches, or, in other words branches that are not sub-branches of other branches. Instead, first-level branches may be any branch in a navigation menu tree that includes one or more sub-branches. In the example of FIG. 6, both Information and Settings branch 124 and Device Information 130 may be considered first-level branches, in spite of the fact that Device Information branch 130 is a sub-branch of Information and Settings branch 124.

With reference to navigation menu tree 120 of FIG. 6, in one example, data transfer module 92 may be configured to detect an active branch, e.g. a branch of the tree currently being accessed by the clinician via user interface 82 of external programmer 20. For example, the clinician may select Information and Settings branch 124 through user interface 82, e.g., by clicking a touch-screen display of programmer 20. Data transfer module 92 may monitor user interaction with user interface 82, and detect when the clinician accesses Information and Settings branch 124. In one example, data transfer module 92 may then be configured to predict a future request for data stored on IMD 13 based on the active branch in navigation menu tree 120 of user interface 82 of external programmer 20. In one example, data transfer module 92 may then be configured to predict the future request for data stored on the IMD 12 that is related to one or more branches in navigation menu tree 120 adjacent to the active Information and Settings branch 124. In such examples, once the clinician navigates to Information and Settings branch 124 of menu navigation tree 120, the chances of the user wanting to view data, e.g., images under Patient information branch 128 may be far greater than their desire to view data under Event Log branch 132, which may not be accessible directly from Information and Settings branch 124. Therefore, data transfer module 92 may be configured to predict a future request for data that is related to a branch in navigation menu tree 120 adjacent to the active Information and Settings branch 124. For example, data transfer module 92 may be configured to predict the future request for data stored on the IMD 12 that is related to a sub-branch of the active Information and Settings branch 124 in navigation menu tree 120, e.g. Patient information branch 128 or Device Information branch 130.

In another example, data transfer module 92 may be configured to predict a future request for data stored on the IMD 13 based on a data request profile for a population of users. A data request profile may include data regarding user behavior in a particular user interface environment. In the example of FIG. 6, the data request profile may include data regarding the manner and order in which a user or a group of users accesses various branches 122 of navigation menu tree 120 presented via user interface 82 of programmer 20. A data request profile for a population of users may include data about user behavior in a particular user interface environment gathered from anecdotal or experimental evidence of the behavior of a number of individual users in a particular category, e.g. a number of individual clinicians or a number of individual patients. Additional information that may be included in data request profiles includes, e.g., user age and/or geographical location.

In one example, data transfer module 92 may be configured to predict a future request for data stored on the IMD 13 by a clinician based on a data request profile for a population of clinicians, which may be stored, e.g. in memory 86 of programmer 20. In one example, data transfer module 92 may predict a future request for data that is related to a branch in navigation menu tree 120 that the data request profile for the population of clinicians indicates has the highest probability of being requested after the currently active branch. For example, the data request profile employed by data transfer module 92 may indicate that in the case Therapy Usage branch 126 is the active branch accessed by the clinician, data under Program Changes branch 132 is most commonly viewed after accessing this branch, followed by data under Event Logs branch 134, then under Amplitude Changes branch 136, and lastly Therapy On/Off transitions branch 138. In this example, data transfer module 92 may rank the order with which to predictively background transfer data from IMD 13 to programmer 20 based on this order indicated by data request profile for the population of clinicians. The data request profile for the population of clinicians may also indicate that after checking data under System Integrity branch 140, if the clinician navigates to Therapy Usage branch 126, they are most likely to check data under Therapy On/Off Transitions branch 138 as part of a troubleshooting technique. As such, in this case, data transfer module 92 may increase the rank of data under Therapy On/Off Transitions branch 138 for background data transfer to the first position after the clinician selects Therapy Usage branch as the active branch.

In some examples, the data request profiles for populations of users employed by data transfer module 92 of programmer 20 to predict future data requests may be dynamically updated by communication between the programmer and another device, e.g. via a local or remote wired or wireless network connection. For example, programmer 20 may be configured to connect to a central database via telemetry module 88 or by another means, e.g. via a wired connection to a local or wide area network. The central database may house data request profiles for various populations of users, which may be periodically updated with new data. Programmer 20 may periodically check the central database for changes to data request profiles, and, if updates have occurred, download the updated profiles to memory 86 from the remote database. The central database, the connection between programmer 20 and the database, and the network infrastructure for such a system may be implemented, in some aspects, with general network technology and functionality similar to that provided by the Medtronic CareLink® Network developed by Medtronic, Inc., of Minneapolis, MN.

In another example, data transfer module 92 may be configured to predict a future request for data stored on the IMD 13 based on a data request profile for a particular user of programmer 20 and/or IMD 13. For example, data transfer module 92 may be configured to predict a future request for data stored on the IMD 13 based on a data request profile for the clinician treating the patient in whom IMD 13 is implanted. Data transfer module 92 may be configured, in such an example, to identify interaction with programmer 20 by this particular clinician based on a user ID and/or other user credentials entered into programmer 20, e.g. stored in memory 86. Data transfer module 92 may generate the data request profile for the clinician treating the patient in whom IMD 13 is implanted by tracking the clinician's use of programmer 20 over time and storing data related to such interactions in memory 86.

In another example, the clinician may manually generate their own data request profile by entering navigation preferences/data access preferences into programmer 20 via user interface 82 for storage on memory 86. However the data request profile is generated for the clinician, data transfer module 92 may be configured to predict a future request for data stored on the IMD 13 by a clinician based on the data request profile for the clinician treating the patient in whom IMD 13 is implanted in much the same manner described above with reference to data request profiles for entire populations of users. For example, data transfer module 92 may predict a future request for data that is related to a branch in navigation menu tree 120 that the data request profile for the particular clinician indicates has the highest probability of being requested after the currently active branch.

In another example, data transfer module 92 may be configured to predict a future request for data stored on the IMD 13 based on a data request profile for the patient in whom IMD 13 is implanted. In such examples, data transfer module 92 may generate the data request profile for the patient in whom IMD 13 is implanted by tracking the patient's use of programmer 20 over time and storing data related to such interactions in memory 86. In another example, the patient may manually generate their own data request profile by entering navigation preferences/data access preferences into programmer 20 via user interface 82 for storage on memory 86. Example information that may be included in the data request profile for a particular user, e.g. clinician or patient, of programmer 20 may include user age and/or geographical location. In one example, the data request profile may be a hybrid of a profile for a particular user and a population of users. For example, data transfer module 92 may be configured to predict a future request for data stored on the IMD 13 based on a data request profile for the patient in whom IMD 13 is implanted, or the clinician treating the patient, which profile may include the personal preferences of that patient or clinician and preferences of a population of users with similar ages and geographical locations as the patient or clinician.

In addition to the foregoing techniques for predicting future data requests, data transfer module 92 may be configured to set or modify data request predictions based on time, e.g., based on a period of time after a clinician visit with a patient begins or after IMD 13 has been implanted within a patient. For example, it may be common for users to access data under Therapy On/Off Transitions branch 138 of navigation menu tree 120 more often for a period of time after IMD 13 has been implanted, because the clinician may not yet be familiar with programmer 20, and may accidentally turn off therapy. However, after some longer period of use, data under Event Logs branch 134 related to, e.g., system crashes may be more commonly viewed. In another example, data transfer module 92 may be configured to set or modify data request predictions based on a period of time after a clinician visit with a patient begins. For example, data under Electrode-to-Electrode Impedance Measurements branch 142 may have a higher probability of being requested by a clinician within a time period at the beginning of a visit, e.g. within the first 3 minutes, while other data, e.g. under Amplitude Changes branch 136 may have a higher probability of being requested by the clinician after a certain time period, e.g. after the first 3 minutes. In this case, data transfer module 92 may be configured to monitor user interface 82 of programmer 20 for initiation of programming or other user of the device by a user, e.g. a clinician. Data transfer module 92, or another component of programmer 20, may be configured to track the time of the user session with programmer 20.

In one example, data transfer module 92 may predict that data under Electrode-to-Electrode Impedance Measurements branch 142 will be selected by the clinician over data under Amplitude Changes branch 136 if the user session with programmer 20 is within, e.g. 3 minutes of beginning. In the event, however, the user session with programmer 20 is more than 3 minutes from the beginning of the session, data transfer module 92 may predict that data under Amplitude Changes branch 136 will be selected by the clinician over data under Electrode-to-Electrode Impedance Measurements branch 142.

In another example, data transfer module 92 may be configured to set or modify data request predictions based on time, e.g., based on the day of the week. For example, a user data request profile may vary during the week versus on the weekend. In one example, access to functions of menu navigation tree 120 by a clinician on a week day, may result in data transfer module 92 predicting that a routine clinician visit may be more likely than, e.g. if the session occurs on the weekend or holiday, which may be associated with a more urgent situation, e.g. an emergency room visit. Such timing may affect the functions that data transfer module 92 predicts are more likely to be accessed by the user.

In another example, data transfer module 92 may predict future data requests based on instrument type or user interface modes. For example, while both clinician and patient programmers can view data under Electrode-to-Electrode Impedance Measurements branch 142 and data under Battery Voltage reading 144, clinician programmers currently accessing System Integrity branch 140 may be more likely to view data under Electrode-to-Electrode Impedance Measurements branch 142, while patient programmers may be more likely to view data under Battery Voltage reading 144. In another example, user interface 82 of programmer 20 may include multiple interaction modes, e.g. a basic mode and an advanced mode. In such an example, data transfer module 92 may predict future data requests based on the current mode of user interface 82 of programmer 20. For example, a patient interacting with user interface 82 of programmer 20 in a basic mode and currently accessing Device Information branch 130 may be more likely to view data under Serial Number branch 146, while a patient employing an advanced mode may be more likely to view data under Lead Type branch 148.

In addition to or in lieu of one or more of the foregoing prediction techniques, data transfer module 92 may make future data request predictions based on the state of the IMD. In one example, data transfer module 92 may predict future data requests based on the power level of power source 34. For example, if the power level of power source 34 is below a threshold level, data transfer module 92 may predict that a user may be likely to navigate to a battery status branch of navigation menu tree 120. In another example, data transfer module 92 may predict future data requests based on sensor data of one or more sensors included in or communicating with IMD 13. For example, IMD 13 may include or be in communication with a posture sensor, e.g. a three-axis accelerometer, which is configured to detect a posture state of the patient in whom IMD 13 is implanted. In one example, if the posture sensor of IMD 13 indicates that the patient just made a posture change or are is currently in a particular posture, data transfer module 92 may predict that the patient, or a clinician may be likely to make a therapy change, e.g. increasing or decreasing stimulation intensity via particular menu branch of navigation menu tree 120 employed for therapy intensity adjustments. In another example, data transfer module 92 may predict future data requests based on sensor or other data from a volume detection mechanism of IMD 15 of FIG. 3, which is configured to indicate the level of therapeutic fluid in reservoir 58 of IMD 15. For example, in the event a volume sensor or other mechanism configured to detect fluid volume in reservoir 58 indicates that the reservoir of IMD 15 is low, data transfer module 92 may predict that a user is likely to check reservoir level status via a volume gauge displayed on programmer 20. Other states of an IMD may be employed in examples according to this disclosure as a basis for predicting data requests, including, e.g. a detected lead integrity compromise may be the basis for predicting that a user may navigate to menu branches of navigation menu tree 120 related to lead integrity.

In some examples of the method of FIG. 5, data transfer module 92 may be configured to combine one or more of the foregoing techniques to predict future data requests that may be made by a user of programmer 20. For example, data transfer module 92 may be configured to predict a future request for data stored on the IMD 13 that is related to one or more branches in navigation menu tree 120 adjacent to the currently active branch and based on a user request profile for a population of users and/or for a particular user of programmer 20.

In addition to predicting a future request for data stored on the IMD (100), the method of FIG. 5 includes detecting that a telemetry session between an IMD and an external device includes available bandwidth (102). In one example, detecting that a telemetry session between an IMD and an external device includes available bandwidth (102) includes detecting that the telemetry session is idle. For example, external programmer 20 may be configured as a clinician programmer and employed by a clinician in the course of programming IMD 13. Upon instruction from the clinician, processor 84 of programmer 20 may command telemetry module 88 to initiate a telemetry session with telemetry module 28 of IMD 13. Data transfer module 92 may be communicatively connected to telemetry module 88 of programmer 20 such that data transfer module 92 may detect if and when the telemetry session between IMD 13 and programmer 20 includes available bandwidth (102). For example, data transfer module 92 may actively monitor telemetry module 88 to determine when an active telemetry session with IMD 13 occurs and when such a session becomes idle. In another example, processor 84 or telemetry module 88 may "wake-up" data transfer module 92 upon initiation of a telemetry session between IMD 13 and programmer 20, after which data transfer module 92 may detect when the telemetry session becomes idle.

The example method of FIG. 5 also includes automatically transferring data that is predicted to be requested in the future from the IMD to the external device via the available bandwidth of the telemetry session (104). For example, after making the future data request prediction employing one or more of the foregoing techniques, data transfer module 92 of programmer 20 may automatically transfer data that the module predicted will be requested in the future from IMD 13 to programmer 20 while the telemetry session is idle and in advance of any request for the data from the user. Data transfer module 92 may be configured to execute the automatic predictive data transfer transparently to the user in the background while the active telemetry session between telemetry module 28 of IMD 13 and telemetry module 88 of programmer 20 is idle.

In some cases, the automatic background data transfer executed by data transfer module 92 may be interrupted by an actual request from a user of programmer 20 via user interface 82. In such an example, data transfer module 92 may be configured to cease the automatic background transfer of data and initiate the transfer from IMD 13 to programmer 20 of the data actually requested by the user. In one example, data transfer module 92 may abandon the automatic background transfer of data such that any data already transferred from IMD 13 to programmer 20 may be disposed of and any future requests for the same data may require starting the transfer over. In another example, data transfer module 92 may pause the automatic background transfer of data such that any data already transferred from IMD 13 to programmer 20 may be retained, e.g., stored in memory 86 or in a hardware stack and after the transfer of the data requested by the user is complete data transfer module 92 may reinitiate the background transfer automatically from the point at which the transfer was previously paused.

In one example, before executing the automatic background transfer, data transfer module 92 may determine if the data that is predicted to be requested in the future was previously transferred from IMD 13 to external programmer 20. In the event the data was previously transferred between the devices, data transfer module 92 may determine if the data on IMD 13 has changed since the previous transfer. For example, data transfer module 92 may communicate with IMD 13 and compare the version of the predicted data on the IMD to the version of the data on programmer 20 from the previous transfer. In the event the data has not changed since the previous transfer, data transfer module 92 may abort the automatic predictive background data transfer, or may execute another prediction algorithm or other determination to predict different data that may be requested by the user of programmer 20 in the future.

Although the target therapy delivery site described with reference to the foregoing examples is proximate to the spinal cord of a patient, other applications of therapy systems in accordance with this disclosure include alternative delivery sites. In some examples, the target delivery site may be proximate to different types of tissues including, e.g., nerves, e.g. sacral, pudendal or perineal nerves, organs, muscles or muscle groups. In one example, a catheter may be positioned to deliver a therapeutic agent or a lead may be position to deliver stimulation to a deep brain site or within the heart or blood vessels. Delivery of a therapy within the brain may help manage a number of disorders or diseases including, e.g., chronic pain, diabetes, depression or other mood disorders, dementia, obsessive-compulsive disorder, migraines, obesity, and movement disorders, such as Parkinson's disease, spasticity, and epilepsy. A catheter may also be positioned to deliver insulin to a patient with diabetes. In other examples, the system may deliver a therapeutic agent or stimulation to various sites within a patient to facilitate other therapies and to manage other conditions including peripheral neuropathy or post-operative pain mitigation, ilioinguinal nerve therapy, intercostal nerve therapy, gastric drug induced stimulation for the treatment of gastric motility disorders and/or obesity, and muscle stimulation, or for mitigation of peripheral and localized pain e.g., leg pain or back pain.

The techniques described in this disclosure for predictive background data transfer from an IMD to an external device may be implemented, at least in part, in hardware, software, firmware or any combination thereof. For example, various aspects of the described techniques may be implemented within one or more processors, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit comprising hardware may also perform one or more of the techniques of this disclosure.

Such hardware, software, and firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

The techniques described in this disclosure may also be embodied or encoded in a computer-readable medium, such as a computer-readable storage medium, containing instructions. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

Various examples have been described. These and other examples are within the scope of the following claims.

## Claims

1. A method comprising:
predicting a future request for data stored on an implantable medical device (IMD);
detecting that a telemetry session between the IMD and an external device comprises available bandwidth; and **characterized by**
automatically transferring data that is predicted to be requested in the future from the IMD to the external device via the available bandwidth of the telemetry session.

2. The method of claim 1, further comprising detecting a state of a user interface of the external device, and wherein predicting the future request for data comprises predicting the future request for data stored on the IMD based on the state of the user interface of the external device.

3. The method of claim 2, wherein detecting a state of a user interface of the external device comprises detecting an active branch in a navigation menu tree of the user interface.

4. The method of claim 3, wherein predicting the future request for data comprises predicting the future request for data stored on the IMD based on the active branch in the navigation menu tree of the user interface.

5. The method of claim 4, wherein predicting the future request for data comprises predicting a future request for data stored on the IMD that is related to one or more branches in the navigation menu tree adjacent to the active branch, or wherein predicting the future request for data comprises predicting the future request for data stored on the IMD based on a data request profile for a population of users, or wherein predicting the future request for data comprises predicting the future request for data stored on the IMD based on a data request profile for a user of the external device, or wherein predicting the future request for data comprises predicting the future request for data stored on the IMD based on time.

6. The method of claim 1, further comprising:
monitoring a user interaction with the external device; and
storing data related to the user interaction with the external device in a data request profile for the user.

7. The method of claim 1, further comprising:
determining if the data that is predicted to be requested in the future was previously transferred from the IMD to the external device; and
determining if the data on the IMD has changed since the previous transfer.

8. The method of claim 1, further comprising:
receiving a request for data stored on the IMD from a user while the data that is predicted to be requested in the future is being automatically transferred from the IMD to the external device;
ceasing the automatic transfer of the data that is predicted to be requested in the future; and
transferring the data requested by the user from the IMD to the external device.

9. An implantable medical device (IMD) comprising:
a telemetry module (28) configured to facilitate communications between the IMD and an external device; and **characterized by**
a processor (24) configured to predict a future request for data stored on the IMD, detect that a telemetry session between the IMD and the external device comprises available bandwidth, and automatically transfer data that is predicted to be requested in the future from the IMD to the external device via the available bandwidth of the telemetry session.

10. A system comprising:
an implantable medical device (IMD) (12);
an external device (20) configured to communicate with the IMD; and **characterized by**
a processor (24, 84) configured to predict a future request for data stored on the IMD, detect that a telemetry session between the IMD and the external device comprises available bandwidth, and automatically transfer data that is predicted to be requested in the future from the IMD to the external device via the available bandwidth of the telemetry session.

11. The system of claim 10, wherein the external device comprises a recharging device configured to recharge a power source of the IMD.

12. A system comprising:
means for predicting a future request for data stored on an implantable medical device (IMD);
means for detecting that a telemetry session between the IMD and an external device comprises available bandwidth; and **characterized by**
means for automatically transferring data that is predicted to be requested in the future from the IMD to the external device via the available bandwidth of the telemetry session.

13. A computer-readable storage medium storing instructions configured to cause a programmable processor to:
predict a future request for data stored on an implantable medical device (IMD);
detect that a telemetry session between the IMD and the external device comprises available bandwidth; and **characterized by** the instructions configured to cause the processor to
automatically transfer data that is predicted to be requested in the future from the IMD to the external device via the available bandwidth of the telemetry session.

14. An external programming device comprising:
a telemetry module (88) configured to facilitate communications between the external programming device and an implantable medical device (IMD); and
a processor (84) configured to predict a future request for data stored on the IMD, detect that a telemetry session between the IMD and the external device comprises available bandwidth, and **characterized by** the processor being configured to automatically transfer data that is predicted to be requested in the future from the IMD to the external device via the available bandwidth of the telemetry session.

## Patentansprüche

1. Verfahren, das umfasst:
Vorhersagen einer zukünftigen Anforderung von Daten, die in einer implantierbaren medizinischen Vorrichtung (IMD) gespeichert sind; und
Detektieren, dass eine Telemetriesitzung zwischen der IMD und einer externen Vorrichtung eine verfügbare Bandbreite enthält; **gekennzeichnet durch**
automatisches Übertragen von Daten, von denen vorhergesagt wird, dass sie in der Zukunft von der IMD angefordert werden, über die verfügbare Bandbreite der Telemetriesitzung an die externe Vorrichtung.

2. Verfahren nach Anspruch 1, das ferner umfasst, einen Zustand einer Anwenderschnittstelle der externen Vorrichtung zu detektieren, wobei das Vorhersagen der zukünftigen Anforderung von Daten umfasst, die zukünftige Anforderung von in der IMD gespeicherten Daten aufgrund des Zustands der Anwenderschnittstelle der externen Vorrichtung vorherzusagen.

3. Verfahren nach Anspruch 2, wobei das Detektieren eines Zustands einer Anwenderschnittstelle der externen Vorrichtung umfasst, einen aktiven Zweig in einem Navigationsmenübaum der Anwenderschnittstelle zu detektieren.

4. Verfahren nach Anspruch 3, wobei das Vorhersagen der zukünftigen Anforderung von Daten umfasst, die zukünftige Anforderung von in der IMD gespeicherten Daten aufgrund des aktiven Zweigs in dem Navigationsmenübaum der Anwenderschnittstelle vorherzusagen.

5. Verfahren nach Anspruch 4, wobei das Vorhersagen der zukünftigen Anforderung von Daten umfasst, eine zukünftige Anforderung von in der IMD gespeicherten Daten, die sich auf einen oder mehrere Zweige in dem Navigationsmenübaum angrenzend an den aktiven Bereich beziehen, vorherzusagen, oder wobei das Vorhersagen der zukünftigen Anforderung von Daten umfasst, die zukünftige Anforderung von in der IMD gespeicherten Daten aufgrund eines Datenanforderungsprofils für eine Anwenderbevölkerung vorherzusagen, oder wobei das Vorhersagen der zukünftigen Anforderung von Daten umfasst, die zukünftige Anforderung von in der IMD gespeicherten Daten aufgrund eines Datenanforderungsprofils eines Anwenders der externen Vorrichtung vorherzusagen, oder wobei das Vorhersagen der zukünftigen Anforderung von Daten umfasst, die zukünftige Anforderung von in der IMD gespeicherten Daten aufgrund der Zeit vorherzusagen.

6. Verfahren nach Anspruch 1, das ferner umfasst:
Überwachen einer Anwenderwechselwirkung mit der externen Vorrichtung; und
Speichern der Daten, die sich auf die Anwenderwechselwirkung mit der externen Vorrichtung beziehen, in einem Datenanforderungsprofil für den Anwender.

7. Verfahren nach Anspruch 1, das ferner umfasst:
Bestimmen, ob die Daten, von denen vorhergesagt wird, dass sie in der Zukunft angefordert werden, vorher von der IMD an die externe Vorrichtung übertragen wurden; und
Bestimmen, ob sich die Daten in der IMD seit der vorherigen Übertragung geändert haben.

8. Verfahren nach Anspruch 1, das ferner umfasst:
Empfangen einer Anforderung von in der IMD gespeicherten Daten von einem Anwender, während die Daten, von denen vorhergesagt wird, dass sie in der Zukunft angefordert werden, automatisch von der IMD an die externe Vorrichtung übertragen werden;
Beenden der automatischen Übertragung der Daten, von denen vorhergesagt wird, dass sie in der Zukunft angefordert werden; und
Übertragen der durch den Anwender angeforderten Daten von der IMD an die externe Vorrichtung.

9. Implantierbare medizinische Vorrichtung (IMD), die enthält:
ein Telemetriemodul (28), das konfiguriert ist, Kommunikationen zwischen der IMD und einer externen Vorrichtung zu erleichtern; und **gekennzeichnet durch**
einen Prozessor (24), der konfiguriert ist, eine zukünftige Anforderung von in der IMD gespeicherten Daten vorherzusagen, zu detektieren, dass eine Telemetriesitzung zwischen der IMD und der externen Vorrichtung eine verfügbare Bandbreite enthält, und Daten, von denen vorhergesagt wird, dass sie in der Zukunft von der IMD angefordert werden, automatisch an die externe Vorrichtung über die verfügbare Bandbreite der Telemetriesitzung zu senden.

10. System, das enthält:
eine implantierbare medizinische Vorrichtung (IMD) (12);
eine externe Vorrichtung (20), die konfiguriert ist, mit der IMD zu kommunizieren; und **gekennzeichnet durch**
einen Prozessor (24, 84), der konfiguriert ist, eine zukünftige Anforderung von in der IMD gespeicherten Daten vorherzusagen, zu detektieren, dass eine Telemetriesitzung zwischen der IMD und der externen Vorrichtung eine verfügbare Bandbreite umfasst, und Daten, von denen vorhergesagt wird, dass die in der Zukunft von der IMD angefordert werden, automatisch über die verfügbare Bandbreite der Telemetriesitzung an die externe Vorrichtung zu senden.

11. System nach Anspruch 10, wobei die externe Vorrichtung eine Ladevorrichtung enthält, die konfiguriert ist, eine Leistungsquelle der IMD zu laden.

12. System, das enthält:
Mittel zum Vorhersagen einer zukünftigen Anforderung von auf einer implantierbaren medizinischen Vorrichtung (IMD) gespeicherten Daten;
Mittel zum Detektieren, dass eine Telemetriesitzung zwischen der IMD und einer externen Vorrichtung eine verfügbare Bandbreite enthält; und **gekennzeichnet durch**
Mittel zum automatischen Übertragen von Daten, von denen vorhergesagt wird, dass sie in der Zukunft von der IMD angefordert werden, an die externe Vorrichtung über die verfügbare Bandbreite der Telemetriesitzung.

13. Computerlesbares Speichermedium, das Anweisungen speichert, die konfiguriert sind, zu bewirken, dass ein programmierbarer Prozessor
eine zukünftige Anforderung von in einer implantierbaren medizinischen Vorrichtung (IMD) gespeicherten Daten vorhersagt;
detektiert, dass eine Telemetriesitzung zwischen der IMD und der externen Vorrichtung eine verfügbare Bandbreite enthält; und **dadurch gekennzeichnet, dass** die Anweisungen konfiguriert sind, zu bewirken, dass der Prozessor
Daten, von denen vorhergesagt wird, dass sie in der Zukunft von der IMD angefordert werden, automatisch über die verfügbare Bandbreite der Telemetriesitzung an die externe Vorrichtung überträgt.

14. Externe Programmiervorrichtung, die enthält:
ein Telemetriemodul (88), das konfiguriert ist, Kommunikationen zwischen der externen Programmiervorrichtung und einer implantierbaren medizinischen Vorrichtung (IMD) zu vereinfachen; und
ein Prozessor (84), der konfiguriert ist, eine zukünftige Anforderung von auf der IMD gespeicherten Daten vorherzusagen, zu detektieren, dass eine Telemetriesitzung zwischen der IMD und der externen Vorrichtung eine verfügbare Bandbreite enthält und **dadurch gekennzeichnet, dass** der Prozessor konfiguriert ist, Daten, von denen vorhergesagt wird, dass sie in der Zukunft von der IMD angefordert werden, automatisch über die verfügbare Bandbreite der Telemetriesitzung an die externe Vorrichtung zu übertragen.

## Revendications

1. Procédé comportant :
de prévoir une demande future de données mémorisées sur un canal implantable (IMD) ;
de détecter qu'une session de télémesure entre le dispositif médical implantable IMD et un dispositif externe comporte une bande passante disponible ; et **caractérisé par** le transfert automatique des données dont la demande est prévue dans le futur depuis le dispositif médical implantable IMD vers le dispositif externe via la bande passante disponible de la session de télémesure.

2. Procédé selon la revendication 1, comportant en outre de détecter un état d'une interface utilisateur du dispositif externe, et dans lequel la prévision de la demande future pour des données comporte de prévoir la demande future pour des données mémorisées sur le dispositif médical implantable IMD sur la base de l'état de l'interface utilisateur du dispositif externe.

3. Procédé selon la revendication 2, dans lequel la détection d'un état d'une interface utilisateur du dispositif externe comporte de détecter une branche active dans une arborescence de navigation de l'interface utilisateur.

4. Procédé selon la revendication 3, dans lequel la prévision de la demande future pour des données comporte de prévoir la demande future pour des données mémorisées sur le dispositif médical implantable IMD sur la base de la branche active dans l'arborescence de navigation de l'interface utilisateur.

5. Procédé selon la revendication 4, dans lequel la prévision de la demande future pour des données comporte de prévoir une demande future pour des données mémorisées sur le médical implantable IMD qui est associé à une ou plusieurs branches dans l'arborescence de navigation adjacente à la branche active, ou dans lequel la prévision de la demande future pour des données comporte de prévoir la demande future pour des données mémorisées sur le dispositif médical implantable IMD sur la base d'un profil de demande de données pour une population d'utilisateurs, ou dans lequel la prévision de la demande future pour des données comporte de prévoir la demande future pour des données mémorisées sur le dispositif médical implantable IMD sur la base d'un profil de demande de données pour un utilisateur du dispositif externe, ou dans lequel la prévision de la demande future pour des données comporte de prévoir la demande future pour des données mémorisées sur le dispositif médical implantable IMD en fonction du temps.

6. Procédé selon la revendication 1, comportant en outré
de surveiller une interaction utilisateur avec le dispositif externe ; et
de mémoriser des données associées à l'interaction utilisateur avec le dispositif externe dans un profil de demande de données pour l'utilisateur.

7. Procédé selon la revendication 1, comportant en outre :
de déterminer si les données dont la demande prévue dans le futur ont été précédemment transférées depuis le dispositif médical implantable IMD vers le dispositif externe ; et
de déterminer si des données sur le dispositif médical implantable IMD ont changé depuis le transfert précédent.

8. Procédé selon la revendication 1, comportant en outre :
de recevoir une demande pour des données mémorisées sur le dispositif médical implantable IMD en provenance d'un utilisateur alors que les données dont la demande est prévue dans le futur sont automatiquement transférées depuis le dispositif médical implantable IMD vers le dispositif externe ;
d'interrompre le transfert automatique des données dont la demande est prévue dans le futur ; et
de transférer les données demandées par l'utilisateur depuis le dispositif médical implantable IMD vers le dispositif externe.

9. Dispositif médical implantable (IMD) comportant :
un module de télémesure (28) configuré pour faciliter des communications entre le dispositif médical implantable IMD et un dispositif externe ; et **caractérisé par**
un processeur (24) configuré pour prévoir une demande future pour des données mémorisées sur le dispositif médical implantable IMD, détecter qu'une session de télémesure entre le dispositif médical implantable IMD et le dispositif externe comporte une bande passante disponible, et transférer automatiquement des données dont la demande est prévue dans le futur depuis le dispositif médical implantable IMD vers le dispositif externe via la bande passante disponible de la session de télémesure.

10. Système comportant :
un dispositif médical implantable (IMD) (12) ;
un dispositif externe (20) configuré pour communiquer avec le dispositif médical implantable IMD ; et **caractérisé par** un processeur (24, 84) configuré pour prévoir une demande future pour des données mémorisées sur le dispositif médical implantable IMD, détecter qu'une session de télémesure entre le dispositif médical implantable IMD et le dispositif externe comporte une bande passante disponible, et transférer automatiquement des données dont la demande est prévue dans le futur depuis le dispositif médical implantable IMD vers le dispositif externe via la bande passante disponible de la session de télémesure.

11. Système selon la revendication 10, dans lequel le dispositif externe comporte un dispositif de rechargement configuré pour recharger une source d'alimentation du dispositif médical implantable IMD.

12. Système comportant :
des moyens pour prévoir une demande future pour des données mémorisées sur un dispositif médical implantable (IMD) ;
des moyens pour détecter qu'une session de télémesure entre le dispositif médical implantable IMD et un dispositif externe comporte une bande passante disponible ; et **caractérisé par**
des moyens pour transférer automatiquement des données dont la demande est prévue dans le futur depuis le dispositif médical implantable IMD vers le dispositif externe via la bande passante disponible de la session de télémesure.

13. Support de mémorisation lisible par ordinateur mémorisant des instructions configurées pour amener un processeur programmable à :
prévoir une demande future pour des données mémorisées sur un dispositif médical implantable (IMD) ;
détecter qu'une session de télémesure entre le dispositif médical implantable IMD et le dispositif externe comporte une bande passante disponible ; et **caractérisé par** les instructions configurées pour amener le processeur à transférer automatiquement des données dont la demande est prévue dans le futur depuis le dispositif médical implantable IMD vers le dispositif externe via la bande passante disponible de la session de télémesure.

14. Dispositif de programmation externe comportant :
un module de télémesure (88) configuré pour faciliter des communications entre le dispositif de programmation externe et le dispositif médical implantable (IMD) ; et
un processeur (84) configuré pour prévoir une demande future pour des données mémorisées sur le dispositif médical implantable IMD, détecter qu'une session de télémesure entre le dispositif médical implantable IMD et le dispositif externe comporte une bande passante disponible, et **caractérisé en ce que** le processeur est configuré pour transférer automatiquement des données dont la demande est prévue dans le futur depuis le dispositif médical implantable IMD vers le dispositif externe via la bande passante disponible de la session de télémesure.
